# EUROPEAN PATENT APPLICATION

(11) **EP 2 498 096 A1**
(43) Date of publication of application: **12.09.2012**
(21) Application number: 12165193.9
(22) Date of filing: 20.07.2007
(51) Int. Cl.: G01N 33/74

(54) **Method of detecting and/or measuring hepcidin in a sample**

(30) Priority: 21.07.2006 US 832625 P
(62) Divisional of application: 07810654.9
(71) Applicant: Amgen Inc., Thousand Oaks, CA 91320-1799 (US)
(72) Inventor: Hongyan, Li, Oak Park, CA 91377 (US); Breau, Alan, Newbury Park, CA 91320 (US); Sasu, Barbra, Westlake Village, CA 91362 (US)
(74) Representative: Williams, Aylsa

(57) **Abstract**

Methods of isolating and/or analysing hepcidin by mass spectrometry and methods of quantifying hepcidin are disclosed

## Description

### CROSS REFERENCE TO RELATED APPLICATIONS

This application claims the benefit of U.S. Provisional Application No. 60/832,625, filed July 21, 2006, which is incorporated by reference in its entirety.

### FIELD OF THE INVENTION

The methods disclosed herein relate to isolation of hepcidin, detection of hepcidin, and measurements of hepcidin levels in biological sample. In particular, the methods disclosed herein allow for efficient isolation of hepcidin from a sample and for quantitative measurement of hepcidin levels in the sample.

### BACKGROUND OF THE INVENTION

Iron is an essential trace element required for growth and development of all living organisms. For example, iron is indispensable for DNA synthesis and in a broad range of metabolic processes. Iron metabolism disturbances have been implicated in a number of significant mammalian diseases, including, but not limited to, iron deficiency anemia, hemosiderosis, and the iron overload disease hemochromatosis (Andrews, Ann. Rev, Genomics Hum. Genet. 1:75 (2000); Philpott, Hepatology 35:993 (2002); Beutler et al., Drug-Metab. Dispos. 29:495 (2001)).

Iron content in mammals is regulated by controlling absorption, predominantly in the duodenum and upper jejunum, which is the only mechanism by which iron stores are physiologically controlled (Philpott, Hepatology 35:993 (2002)). Following absorption, iron is bound to circulating transferrin and delivered to tissues throughout the body. The liver is the major site of iron storage.

A feedback mechanism exists that enhances iron absorption in individuals who are iron deficient, and that reduces iron absorption in individuals with iron overload (Andrews Ann. Rev. Genomics Hum. Genet. 1:75 (2000); Philpott, Hepatology 35:993(2002); Beutler et al., Drug-Metab. Dispos. 29:495(2001)). The molecular mechanism by which the intestine responds to alterations in body iron requirements have only recently been elucidated. In this context, hepcidin, a recently identified mammalian polypeptide (Krause et al., FEBS Lett, 480:147 (2000); Park et al., J. Biol. Chem. 276:7806 (2001)), has been demonstrated to be a key signaling component regulating iron homeostasis (Philpott, Hepatology 35:993 (2002); Nicolas et al., Proc. Natl. Acad. Sci. USA 99:4396 (2002)). Hepcidin was isolated as a 25 amino acid (aa) polypeptide in human plasma and urine, exhibiting antimicrobial activity (Krause et al., FEBS Lett. 480:147 (2000); Park et al., J. Biol. Chem. 276:7806 (2001)). A hepcidin cDNA encoding an 83 aa precursor in mice and an 84 aa precursor in rat and human, including a putative 24 aa signal peptide, were subsequently identified searching for liver specific genes that were regulated by iron (Pigeon et al., J. Biol. Chem. 276:7811 (2001)).

The association of hepcidin with innate immune response derives from the observation of a robust upregulation of hepcidin gene expression after inflammatory stimuli, such as infections, which induce the acute phase response of the innate immune systems of vertebrates. In mice, hepcidin gene expression was shown to be upregulated by lipopolysaccharide (LPS), turpentine, Freund's complete adjuvant, and adenoviral infections.

Studies conducted with human primary hepatocytes indicated that hepcidin gene expression responded to the addition of interleukin-6 (IL-6), but not to interleukin-1α (IL-1α) or tumor necrosis factor-α (TNF-α). Concordant with this observation, infusion of human volunteers with IL-6 caused the rapid increase of bioactive hepcidin peptide levels in serum and urine, and was paralleled by a decrease in serum iron and transferrin saturation. A strong correlation between hepcidin expression and anemia of inflammation also was found in patients with chronic inflammatory diseases, including bacterial, fungal, and viral infections. These findings, in association with similar murine data, led to the conclusion that induction of hepcidin during inflammation depends on IL-6, and that the hepcidin-IL-6 axis is responsible for the hypoferremic response and subsequent restriction of iron from blood-borne pathogens.

The central role of hepcidin and its key functions in iron regulation and in the innate immune response to infection illustrates the need for methods and informative diagnostic tools for the measurement of mature, bioactive forms of hepcidin in biological samples and for the regulation of hepcidin production.

By partially purifying hepcidin from urine samples, it was demonstrated that hepcidin secretion into urine is increased between 10 and 100-fold relative to normal levels in conditions of inflammation (Park et al., J. Biol Chem. 276(11):7806 (2001)). In mouse studies, the only method of detecting hepcidin up-regulation relied on RNA analysis, which may not correlate to circulating hepcidin levels. To date, no method is available to accurately measure levels of hepcidin in serum or plasma. The urinary method described above does not shed light on absolute circulating hepcidin levels. Other assays developed to measure serum levels are either semi-quantitative (Tomosugi et al., Blood, April 2006 prepublication), or only capable of detecting pro-hepcidin, a species which does not correlate with hepcidin induction (Kemna et al., Blood 106:1864-1866 (2005)). An assay to allow accurate quantification of hepcidin levels is critical to identify patients who may benefit from a hepcidin-blocking strategy to treat the anemia of inflammation or other related disorders.

### SUMMARY OF THE INVENTION

Disclosed herein are methods of isolating and/or determining the concentration of hepcidin in a sample. In particular, a method for quantifying hepcidin in a biological sample using mass spectrometry and a method of purifying and/or separating hepcidin from a biological sample are disclosed.

Therefore, one aspect of the invention is to provide a method of determining the presence or concentration of hepcidin in a sample comprising subjecting the sample to mass spectrometry (MS) to produce a mass spectrum having a signal corresponding to hepcidin; measuring the intensity of the signal; and correlating the signal intensity to a standard curve of hepcidin concentrations. In some embodiments, the hepcidin is a prepropeptide form of hepcidin having about 84 amino acid residues. In other embodiments, the hepcidin is a propeptide form of hepcidin having about 61 amino acid residues. In still other embodiments, the hepcidin is a processed form of hepcidin having about 20 to about 25 amino acid residues. In some embodiments, the hepcidin is a peptide having a sequence of SEQ. ID NO: 3; SEQ. ID NO: 4; SEQ. ID NO: 13; SEQ. ID NO: 14; SEQ. ID NO: 15, or a combination thereof. The hepcidin is ionized during MS analysis, and the resulting charge of hepcidin can be + 1, +2, +3, +4, or a mixture thereof. The MS analysis can be through liquid chromatography-MS and/or tandem MS. In some embodiments, the hepcidin is further separated from the sample prior to MS analysis. Separation can occur through chromatography, such as liquid chromatography and solid phase extraction.

Another aspect of the invention is to provide a method of determining the presence or concentration of hepcidin in a sample comprising separating the hepcidin from a sample; subjecting the hepcidin to MS to produce a mass spectrum having a signal corresponding to hepcidin; measuring the intensity of the hepcidin signal; and correlating the signal intensity in the mass spectrum to a standard curve of hepcidin concentrations to obtain a quantity of hepcidin in the sample. In some embodiments, the separating of hepcidin from the sample is through chromatography, such as liquid chromatography, solid phase extraction, or a combination thereof. In some embodiments, the hepcidin is a preproteptide form of hepcidin having about 84 amino acid residues. In other embodiments, the hepcidin is a propeptide form of hepcidin having about 61 amino acid residues. In still other embodiments, the hepcidin is a processed form of hepcidin having about 20 to about 25 amino acid residues. The hepcidin is ionized during MS analysis, and the resulting charge of hepcidin can be +1, +2, +3, +4, or a mixture thereof.

Yet another aspect of the invention is to provide a method of separating hepcidin from a sample, comprising introducing the sample to a reverse phase column and treating the reverse phase column with an eluting solvent, wherein the resulting eluant comprises hepcidin. In some embodiments, the eluting solvent comprises methanol, water, or a mixture thereof. In certain embodiments, the reverse phase column comprises a C18, C8, or C3 reverse phase column or polar modified C18, C8, or C3 reverse phase column. Such columns are available from a variety of commercial sources, including Waters, Agilent, and the like.

In any of the aspects of the invention disclosed herein, the sample can be from a mammal, and in specific embodiments, the mammal is human. In certain embodiments, the human is healthy, while in other embodiments, the human exhibits disrupted iron homeostasis. In specific embodiments, the iron homeostasis is below normal, while in other embodiments, the iron homeostasis is above normal. In one specific embodiment, iron indices or hematological criteria of the subject are outside normal ranges as indicated in Table I, below. In various embodiments, a human patient suffers from anemia and is hypo-responsive to erythropoietin therapy or an erythropoietic therapy. In a specific embodiment, the erythropoietic therapy comprises an erythropoietin analog such as darbepoetin alfa.

In any of the aspects of the invention disclosed herein, the human may suffer from or be suspected of suffering from an inflammatory or inflammatory-related condition. Such conditions include sepsis, anemia of inflammation, anemia of cancer, chronic inflammatory anemia, congestive heart failure, end stage renal disease, iron deficiency anemia, ferroportin disease, hemochromatosis, diabetes, rheumatoid arthritis, arteriosclerosis, tumors, vasculitis, systemic lupus erythematosus, and arthopathy. In other embodiments, the human may suffer from or is suspected of suffering from a non-inflammatory condition. Such non-inflammatory conditions include vitamin B6 deficiency, vitamin B12 deficiency, folate deficiency, pellagra, funicular myelosis, pseudoencephalitis, Parkinson's disease, Alzheimer's disease, coronary heart disease and peripheral occlusive arterial disease. In still other embodiments, the human may suffer from or is suspected of suffering from an acute phase reaction. Such acute phase reactions include sepsis, pancreatitis, hepatitis and rheumatoid diseases.

In another aspect of the invention, methods are provided for improving treatment of a human patient suffering from anemia by measuring hepcidin concentrations using the methods disclosed herein. In various embodiments, the hepcidin concentration is about 10 ng/mL or less, about 5 ng/mL or less, about 2 ng/mL or less, or about 1 ng/mL or less. In other embodiments, the hepcidin concentration is about 25 ng/mL or greater or 30 ng/mL or greater. In certain embodiments, the patient's anemia therapy is modified and/or reassessed based upon monitoring of his or her hepcidin concentration.

Another aspect of the invention is a kit comprising two or more items useful for practicing a method of the invention, packaged together. For example, in one variation, the kit comprises a plurality of hepcidin containers, each container having a known and different amount of hepcidin, a standard container, and instructions for preparing a standard curve of hepcidin concentrations to standard and for assaying a test sample for hepcidin concentration. In certain cases, the standard comprises an isotopically labeled hepcidin, while in other cases, the standard is a peptide which has similar retention as hepcidin and a mass about ± 750 Da of that of hepcidin. In some embodiments, the hepcidin comprises SEQ. ID NO: 4. In a specific embodiment, the hepcidin comprises SEQ. ID NO: 4 and the standard comprises isotopically labeled SEQ. ID NO: 4.

### BRIEF DESCRIPTION OF THE FIGURES

FIG. 1A shows a decision tree of iron indices and disease states for assessment of a patient, absent a measurement of hepcidin levels;

FIG. 1B shows a decision tree for assessment of a patient using measurement of hepcidin levels;

FIG. 2 shows that the formation of hepcidin ions in a mass spectrum is predominantly charge state +4 (m/z 698.5) and +3 (m/z 930.8);

FIG. 3 shows the MS/MS hepcidin spectrum of the +3 charge state;

FIG. 4 shows the MS/MS hepcidin spectrum of the +4 charge state;

FIG. 5 shows pH optimization for solid phase extraction (SPE) of hepcidin;

FIG. 6 shows MS chromatograms of human hepcidin (+3 and +4 charge states) extracted from blank human serum;

FIG. 7 shows MS chromatograms of human hepcidin (+3 and +4 charge states) and internal standard extracted from a sepsis patient;

FIG. 8 shows a standard curve of concentration vs. analyte area : internal standard area created from known hepcidin amounts;

FIG. 9 shows stability of hepcidin, over time under incubation conditions;

FIG. 10 shows a base peak LC chromatogram of an incubation solution at time 0;

FIG. 11 shows the corresponding MS chromatogram of hepcidin at incubation time 0;

FIG. 12 shows the base peak chromatogram of a solution after 4 hours incubation;

FIG. 13 shows the corresponding MS chromatogram of hepcidin at incubation time 4 hours;

FIG. 14A shows comparison of serum hepcidin levels in 20 volunteer donors compared to 40 patients with a variety of cancers and a Hemoglobin less than 10 g/dL, where two elevated readings in the control donors correlated with high Tsat or infection; and

FIG. 14B shows sub-classification of the anemia of cancer patients in order to identify those with probable inflammation (AI), iron deficiency anemia (IDA) or a mixture of the two (mixed anemia). Any patients falling into none of these classes was designated 'other'. Indices used to classify patients were CRP, sTfR/log ferritin ratio, Tsat, and ferritin (as seen in FIG. 1B). Lines represent the mean value.

FIG. 15 shows a MS chromatogram of truncated human hepcidin Hepc-20 (SEQ ID NO: 15), which shows Hepc-20 in both a +3 and +4 charge state.

FIG. 16 shows a MS chromatogram of truncated human hepcidin Hepc-22 (SEQ ID NO: 16), which shows Hepc-22 in both a +3 and +4 charge state.

FIG. 17 shows a MS chromatogram of human hepcidin Hepc-25 (SEQ ID NO: 4), which shows Hepc-25 in both a +3 and +4 charge state.

FIG. 18 shows calibration curves for concentration (ng/mL) of Hepc-25, Hepc-22, and Hepc-20 by intensity of MS signal.

### DETAILED DESCRIPTION

Hepcidin, named because of its site of production, is an important peptide linked to iron metabolism. Disturbances in iron metabolism can be assessed by measuring hepcidin levels, then comparing the measured hepcidin levels to normal levels. Such comparisons can facilitate diagnosis, can assess treatment regimens, or can monitor a patient's progress over time.

The disclosed methods provide a means of isolating hepcidin from and/or analyzing hepcidin in a biological sample. Prior to this disclosure, neither mass spectrometry of nor isolation of hepcidin from a sample was sufficiently efficient or accurate to correlate to an absolute quantification of hepcidin in the sample. Inefficient MS and/or separation techniques, or inconsistent MS and/or separation techniques, hinder one's ability to compare discrete sets of data. With the discovery of efficient MS and separation techniques, various data sets can be compared, both from the same patient over time, or different patients at different times.

Hepcidin, due in part to its amino acid sequence, is not easily fragmented for MS analysis. Disclosed herein are techniques for proficient ionization and fragmentation of hepcidin, which results in MS spectra suitable for quantification of hepcidin using internal standard and standard curve comparisons. Prior reports of hepcidin MS analyses utilized MS techniques which do not effectively permit quantification of hepcidin in a sample. (See, e.g., Kemna et al., Blood, 106:3268 (2005).) The methods disclosed herein also can be applied to measurement of defensins (Kluver et al., J. Peptide. Res. 59:241 (2002)).

As used herein, the terms "quantitative" or "quantification" refer to providing an absolute measurement of hepcidin in a sample that can be compared to measurements taken at a different time or from a different source. Quantitative measurements are valuable for many purposes in addition to relative measurements that only can be compared to other measurements taken at the same time that may yield information such as a ratio. As described below in greater detail, the use of a measured quantity of an internal standard permits quantitative calculation of the hepcidin in a sample.

As used herein, "sample" means any biological sample suitable for hepcidin analysis by the methods disclosed herein. The source of such samples can be serum, blood, plasma, urine, or other bodily fluid, or a filtrate of a bodily fluid, from a test subject. The test subject is an animal, preferably mammal, and more preferably human.

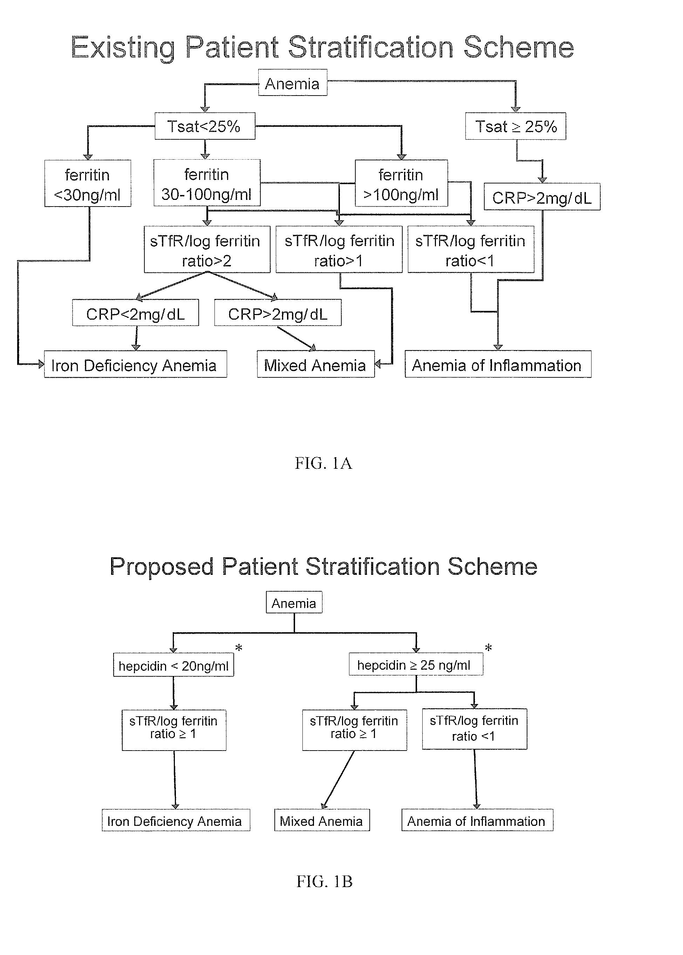

As used herein, an "iron marker" is a metabolite, protein, or other biomolecule which is implicated in iron levels or metabolism. A disruption of iron metabolism signifies a deficiency or deviation from normal values of one or more iron markers, such as those listed in Table I, or other clinical iron parameters, such as those listed in FIG. 1A or FIG. 1B. Proteins, in addition to clinical iron parameters, which may control iron levels include ferroportin, soluble hemojuvelin, bone morphogenic proteins (BMP) and related family members. By measuring hepcidin levels in a biological sample from a patient, information concerning the iron metabolism of the patient can be extrapolated. Further, data concerning trends in hepcidin levels with respect to various iron metabolism disorders or conditions can be generated.

Inflammatory conditions which are implicated in a disruption of iron metabolism include, but are not limited to, sepsis, anemia of inflammation (Weiss et al., N. Engl. J Med. 352:1011 (2005)), anemia of cancer, collagen-induced arthritis (CIA), congestive heart failure (CHF), end stage renal disease (ESRD) (Kulaksiz et al., Gut, 53:735 (2004)), iron deficiency, hemochromatosis (Ganz, Blood, 102(3):783 (2003)), diabetes, rheumatoid arthritis (Jordan, Curr. Opin. Rheumatology, 16:62 (2004)), arteriosclerosis, tumors, vasculitis, systemic lupus erythematosus, and kidney disease or failure. Non-inflammatory conditions which are implicated in a disruption of iron metabolism include, but are not limited to, vitamin B6 deficiency, vitamin. B12 deficiency, folate deficiency, pellagra, funicular myelosis, pseudoencephalitis, Parkinson's disease (Fasano et al., J. Neurochem. 96:909 (2006) and Kaur et al., Ageing Res. Rev., 3:327 (2004)), Alzheimer's disease, coronary heart disease, osteopenia and osteoporosis (Guggenbuhl et al., Osteoporos. Int. 16:1809 (2005)), hemoglobinopathies and other disorders of red cell metabolism (Papanikolaou et al., Blood 105:4103 (2005)), and peripheral occlusive arterial disease. Acute phase reaction conditions which are implicated in a disruption of iron metabolism include, but are not limited to, pancreatitis, hepatitis (Brock, Curr. Opin. Clinic. Nutrition. Metab. Care, 2:507 (1999)), and rheumatoid diseases.

The reference values for the measured hepcidin are, for example, in the range of 0 to about 2000 ng/mL, in particular about 100 to about 500 ng/mL. A particularly preferred reference range is about 200 to about 260 ng/mL. Any value within the reference range can be used as a threshold value for the measurement, for example a value in the range of 0 to about 1000 ng/mL. A measurement below about 10 ng/mL may indicate suppression of hepcidin, while the "normal" range of hepcidin levels is less than about 25 ng/mL. Various other iron indices and their normal ranges of concentrations are listed in Table I.

**Table I**

| **Iron Index** | **Normal Level (Range)** |
|---|---|
| Serum iron | 50-170 µg/dL |
| Hemoglobin | 11.5-18 g/dL |
| Hematocrit | 37-54% |
| Mean Corpuscular Volume (MCV) | 80-96 fL |
| Red Cell Distribution Width (RDW) | 11.5-14.5% (electrical impedence method) or 10.2-11.8% (laser light method) |
| Total Iron Binding Capacity (TIBC) | 250-450 µg/dL |
| Transferrin Iron Saturation Percentage (Tsat) | 15-50% |
| Ferritin | 12-120 µg/L |
| Folate | 3-16 ng/mL (serum) and 130-628 ng/mL (red blood cell) |
| Vitamin B12 | 200-900 µg/ml |

A patient's iron index level outside of the normal ranges listed in Table I is a trigger to measure the hepcidin level of that patient. Because hepcidin is a key component of iron metabolism, hepcidin levels correlate to a disruption of iron metabolism and/or iron indices. Elevated hepcidin levels correlate with serum iron levels below the normal ranges indicated in Table I, low hemoglobin, and hematocrit, reduced or normal Tsat and high or normal ferritin values, and elevated inflammatory status as measured by C-reactive protein (CRP) elevation.

Detection of disruption of iron metabolism includes assaying, imaging, or otherwise establishing the presence, absence, or concentration of hepcidin or a hepcidin precursor. The term "detection" encompasses diagnostic, prognostic, and monitoring applications for hepcidin.

The term "hepcidin," as used herein, includes prepropeptides of 83 or 84 aa sequences in mouse (SEQ. ID NO: 1); rat (SEQ ID NO: 2); and human (SEQ ID NO: 3); the 25 aa hepcidin sequences of human hepcidin (SEQ. ID NO: 4); cyno hepcidin (SEQ. ID NO: 5); vervet monkey hepcidin (SEQ. ID NO: 6); rabbit hepcidin (SEQ. ID NO: 7); rat hepcidin (SEQ. ID NO: 8); mouse hepcidin (SEQ. ID NO: 9); or canine hepcidin (SEQ. ID NO: 10), (SEQ. ID NO: 11), or (SEQ. ID NO: 12). Hepcidin peptides also include a 60 amino acid human propetide (SEQ. ID NO: 13) and (SEQ. ID NO: 14), as well as a 20 amino acid sequence (SEQ ID NO: 15), and 22 amino acid sequence (SEQ ID NO: 16).

The terms "mass spectrometry" or "MS" as used herein refer to methods of filtering, detecting, and measuring ions based on their mass-to-charge ratio, or "*m*/*z*." In general, one or more molecules of interest are ionized, and the ions are subsequently introduced into a mass spectrographic instrument where, due to a combination of magnetic and electric fields, the ions follow a path in space that is dependent upon mass ("m") and charge ("z"). See, e.g., U.S. Pat. No. 6,204,500; 6,107,623; 6,268,144; 6,124,137; 6,982,414; 6,940,065; 5,248,875; Wright et al., Prostate Cancer and Prostatic Diseases 2:264 (1999); and Merchant and Weinberger, Electrophoresis 21:1164 (2000), each of which is hereby incorporated by reference in its entirety.

For example, in a "quadrupole" or "quadrupole ion trap" instrument, ions in an oscillating radio frequency (RF) field experience a force proportional to the direct current (DC) potential applied between electrodes, the amplitude of the RF signal, and *m*/*z.* The voltage and amplitude can be selected such that only ions having a particular *m*/*z* travel the length of the quadrupole, while all other ions are deflected. Thus, quadrupole instruments can act as both a "mass filter" and as a "mass detector" for the ions injected into the instrument.

Triple quadrupole mass spectrometer in multiple ion reaction monitoring (MRM) mode preferably is used for hepcidin quantification. It involves two stand-alone quadrupole mass analyzers, separated by a quadrupole collision cell. The first quadrupole is used to select analyte ions of interest (precursor), which is fragmented in the collision cell by collision-induced dissociation. The resulting fragments are analyzed by the second analytical quadrupole. Because precursor ions break apart in the collision cell very specifically, monitoring the unique fragment ion of the precursor leads to great improvement of the specificity and signal to noise ratio. It therefore can be used for quantitative analysis on complex samples like serum.

Additionally, the resolution of the MS techniques can be enhanced by employing "tandem mass spectrometry," or "MS/MS." In this technique, a precursor ion or group of ions generated from a molecule (or molecules) of interest are filtered in an MS instrument, and these precursor ions subsequently are fragmented to yield one or more fragment ions that then are analyzed in a second MS procedure. By careful selection of precursor ions, only ions produced by certain analytes of interest are passed to the fragmentation chamber, where collision with atoms of an inert gas occurs to produce the fragment ions. Because both the precursor and fragment ions are produced in a reproducible fashion under a given set of ionization/fragmentation conditions, the MS/MS technique can provide an extremely powerful analytical tool. For example, the combination of filtration/fragmentation can be used to eliminate interfering substances, and can be particularly useful in complex samples, such as biological samples.

Mass spectrometers utilize a number of different ionization methods. These methods include, but are not limited to, gas phase ionization sources, such as electron impact, chemical ionization, and field ionization, as well as desorption sources, such as field desorption, fast atom bombardment, matrix assisted laser desorption/ionization, and surface enhanced laser desorption/ionization. In addition, mass spectrometers can be coupled to separation means such as gas chromatography (GC) and high performance liquid chromatography (HPLC). In some cases, electrospray ionization is employed. Electrospray ionization (ESI) in positive ion mode is used to interface the HPLC separation and mass spectrometer detection of hepcidin. Ionization takes place at atmospheric pressure. It involves spraying the effluent of an LC analysis or a sample itself out of a small needle, to which a high voltage is applied. This process produces small charged droplets, and the mobile phase solvent is then evaporated leaving the sample molecule in the gas phase and ionized. These generated gas phase ions then are "swept" into mass spectrometer for detection. Other ionization techniques can be employed as well, such as chemical ionization, but ESI is the preferred ionization mechanism. Because a mass spectrum is reported in m/z units, the degree of ionization will affect the resulting peak corresponding to the hepcidin peptide. The charge of hepcidin can be +1, +2, +3, +4, or a combination thereof, depending upon the ionization method employed.

In some embodiments, the MS is combined with a liquid chromatography step in order to increase sensitivity of the resulting measurements. Reverse phase HPLC is a separation technique involving mass-transfer between two immiscible non-polar stationary and relative polar mobile phases. The components of a mixture first are dissolved in a solvent, then introduced into the mobile phase and flow through a chromatographic column where the stationary phase is immobilized on the packing material. In the column, the mixture is resolved into its components depending upon the interaction of the solute with mobile and stationary phases. Each of the resolved components is then detected by the mass spectrometer.

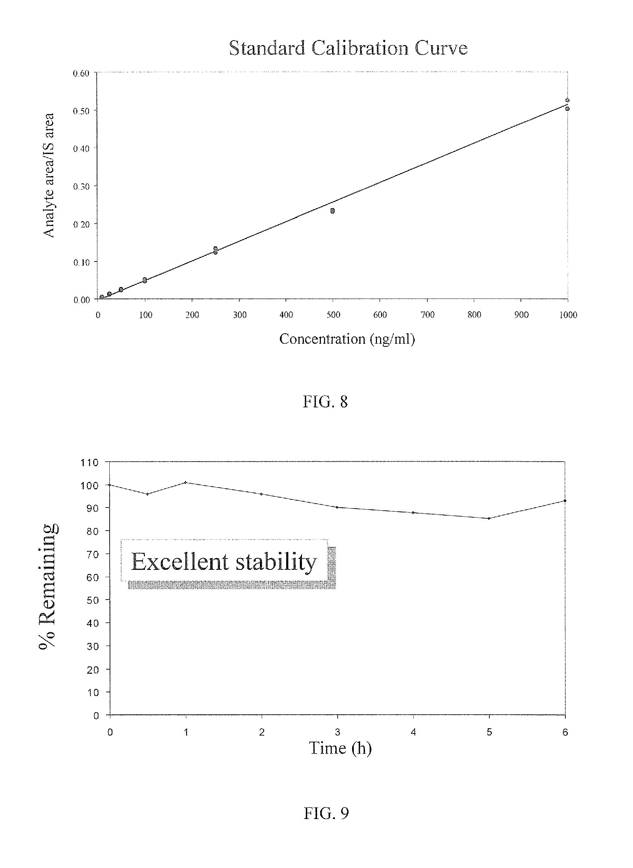

Quantification of hepcidin in a sample is achieved by adding an internal standard to the samples and comparing the ratio of hepcidin and internal standard to ratios obtained from a series of samples where known amounts of hepcidin and internal standard are added to blank matrix (a standard curve, see, e.g., FIG. 8). The use of this method allows for the determination of the levels ofhepcidin in normal healthy individuals, as well as the level of hepcidin from patients suffering sepsis or other inflammatory conditions. Appropriate internal standards are those which have a distinct molecular weight (i.e., mass spectrum signal) from that of hepcidin, but within a ± 750 (m/z) range. In some embodiments, the internal standard is an isotopically labeled hepcidin peptide, wherein one or more H, C, N, and/or O atoms is replaced with a stable isotope having a different mass (e.g., ²H, ¹³C, ¹⁵N, ¹⁷O, or ¹⁸O). In various embodiments, the internal standard is a peptide having similar retention and/or chromatographic characteristics as hepcidin and a distinct molecular weight, within ± 50 m/z. In a specific embodiment, the internal standard is a peptide of SEQ. ID NO: 17.

The quantification of hepcidin levels in a sample permits comparisons between data sets. This ability to compare different sample measurements permits diagnosis as well as monitoring abilities. The quantification methods for hepcidin according to embodiments of the present invention can be advantageously utilized in diagnosing the extent of iron metabolism or iron homeostasis disruption in patients within a clinical diagnostic setting. Such diagnosis could in turn be used by clinicians to select and implement appropriate preventative and treatment therapies including iron chelation, iron treatment, inflammatory suppression, or erythropoietic therapy. In a clinical diagnostic setting, the above methods and calculations are interpreted such that a proper course of action can be taken depending on the concentration levels of hepcidin, alone or in combination with other parameters such as those listed in Table I. As used herein, the term "iron homeostasis" refers to a process of coordinating all aspects of iron metabolism in the body to maintain a balance between iron uptake, iron loss, and iron mobilization and storage, resulting in control of serum iron' levels within the normal range. Iron homeostasis with a negative balance (below normal) would consist of situations where serum iron levels were maintained below the normal range, the balance between iron uptake and loss led to a net iron loss to the body or iron maldistribution occurred such that the iron stores became depleted. Iron homeostasis with a positive balance (above normal) would consist of situations where serum iron levels were maintained above the normal range, the balance between iron uptake and loss led to a net iron gain to the body or iron maldistribution occurred such that the iron stores became increased.

In order to facilitate the diagnosis of patients, decision trees, such as that of FIG. 1B, can be used to interpret the level of the hepcidin, and which is used to assist the user or interpreter in determining a course of treatment and the significance of the concentration reading. Hepcidin values are predicted to be elevated in patients with inflammation iron overload and ferroportin disease and suppressed in patients with hemochromatosis, hemoglobinopathies, and other red cell disorders. The decision tree of FIG. 1B shows how measurement of hepcidin levels simplifies diagnosis and/or assessment of a patient suspected of having iron metabolism disorders. FIG. 1A shows the decision tree assessment without a measurement of hepcidin levels.

In various embodiments, hepcidin levels can be used to improve treatment of a patient with anemia. By analyzing the concentration of hepcidin levels in a patient, the medical decision maker can better evaluate a particular treatment under consideration or currently undertaken by the patient. In particular, patients who are hypo-responsive to typical anemia treatments, such as erythropoietin or analogs thereof (Epoetin alfa, Epoetin beta, darbepoetin alfa, any erythropoietic stimulating protein or peptide or small molecule with erythropoietic stimulating activity), or any other kind of erythropoeitic therapy, benefit from rapid and/or accurate analyses of alternative anemia treatments. In some embodiments, the anemia treatment is tailored for an individual patient based upon monitoring of the patient's hepcidin levels in response to each anemia treatment.

The term "erythropoiesis-stimulating molecules" or "erythropoietic therapy" as used herein includes human erythropoietin or a biologically active variant, derivative, or analog thereof, including a chemically modified derivative of such protein or analog or any small molecule which stimulates erythropoiesis. Erythropoietin includes but is not limited to, a polypeptide comprising the amino acid sequence as set forth in SED. ID NO: 18 or SEQ. ID NO: 19. Amino acids 1 through 165 of SEQ ID NO: 18 constitute the mature protein of any molecules designated as an epoetin, e.g., epoetin alfa, epoetin beta, epotein gamma, epoetin zeta, and the like. Additionally, an epoein also includes any of the aforementioned epoetin which are chemically modified, e.g., with one or more water-soluble polymers such as, e.g., polyethylene glycol. Also contemplated are analogs of erythropoietin, with 65%, 70%, 75%, 80%, 85%, 90%, 91%, 92%, 93%, 94%, 95%, 96%, 97%, 98%, or 99% identity to SEQ. ID NO: 18 or SEQ. ID NO: 19, and still retaining erythropoietic activity.

Exemplary sequences, manufacture, purification and use of recombinant human erythropoietin are described in a number of patent publications, including but not limited to Lin U.S. Patent 4,703,008 and Lai et al. U. S. Patent 4,667,016, each of which is incorporated herein by reference in its entirety. Darbepoetin is a hyperglycosylated erythropoietin analog having five changes in the amino acid sequence of rHuEPO which provide for two additional carbohydrate chains. More specifically, darbepoetin alfa contains two additional N-linked carbohydrate chains at amino acid residues 30 and 88 of SEQ ID NO: 18. Exemplary sequences, manufacture, purification and use of darbepoetin and other erythropoietin analogs are described in a number of patent publications, including Strickland et al., WO 91/05867, Elliott et al., WO 95/05465, Egrie et al., WO 00/24893, and Egrie et al. WO 01//81405, each of which is incorporated herein by reference in its entirety. Derivatives of naturally occurring or analog polypeptides include those which have been chemically modified, for example, to attach water soluble polymer (e.g., pegylated), radionuclides, or other diagnostic or targeting or therapeutic moieties.

The term "erythropoietic activity" means activity to stimulate erythropoiesis as demonstrated in an *in vivo* assay, for example, the exhypoxic polycythemic mouse assay. See, e.g., Cotes and Bangham, Nature 191:1065 (1961).

In various embodiments of the disclosed methods, hepcidin is isolated from a sample prior to, or in place of, MS or LC-MS analysis. Solid phase extraction (SPE) is a preferred means of hepcidin isolation. Use of SPE allows for extraction and isolation of hepcidin from biological sample (such as serum, plasma, and urine) for subsequent HPLC separation and mass spectrometric detection. SPE is a chromatographic technique for preparing samples prior to performing quantitative chemical analysis, such as MS. The goal of SPE is to isolate target analytes from a complex sample matrix containing unwanted interferences, which would have a negative effect on the ability to perform quantitative analysis. The isolated target analytes are recovered in a solution that is compatible with quantitative analysis. This final solution containing the target compound can be used for analysis directly, or evaporated and reconstituted in another solution of a lesser volume for the purpose of further concentrating the target analyte, and making it more amenable to detection and measurement. Analysis of biological samples, such as plasma and urine, using liquid chromatography (LC) generally benefits from SPE prior to analysis both to remove insoluble matter and soluble interferences, and also to pre-concentrate target compounds for enhanced detection sensitivity. Many sample matrices encountered in bio-separations contain buffers, salts, or surfactants, which can be particularly troublesome when mass spectrometer based detection is used. SPE can also be used to perform a simple fractionation of a sample based on differences in the chemical structure of the component parts, thereby reducing the complexity of the sample to be analyzed.

Typical SPE methods contain a sequence of steps, each with a specific purpose. The first step, referred to as the "conditioning" step, prepares the device, typically a chromatography column, for receiving the sample. For reversed-phase SPE, the conditioning step involves first flushing the SPE device with an organic solvent such as methanol or acetonitrile, which acts to wet the surfaces of both the device and the sorbent, and also rinses any residual contaminants from the device. This initial rinse generally is followed by a highly aqueous solvent rinse, often containing pH buffers or other modifiers, which will prepare the chromatographic sorbent to preferentially retain the target sample components. Once conditioned, the SPE device is ready to receive the sample.

The second step, referred to as the "loading" step, involves passing the sample through the device. During loading, the sample components, along with many interferences are adsorbed onto the chromatographic sorbent. Once loading is complete, a "washing" step is used to rinse away interfering sample components, while allowing the target compounds to remain retained on the sorbent. With little or no loss of hepcidin, the resulting measurement ofhepcidin accurately reflects the actual amount of hepcidin in the sample.

For washing of the sample containing hepcidin, a pH of greater than 7 is preferred, a pH of greater than 8 is more preferred, and a pH of about 10 or higher is most preferred. Washing using a solvent system in this range of pH values allows for contaminants and other biomolecules in the sample to be washed away from the hepcidin, with little or no loss of the hepcidin itself. Buffers used to adjust the pH of the washing solvent include, but are not limited to, ammonium hydroxide, phosphate, carbonates, and the like. Ammonium hydroxide is the preferred buffer. The amount of buffer present in the solvent system will affect the resulting pH, but is typically present in amount of about 0.1 to about 10%, more preferably about 1 to about 5%, and most preferably about 1.5 to about 3%. In one specific embodiment, ammonium hydroxide is the buffer and is present in an amount of about 2%. The washing solvent typically is a mixture of water and methanol. Methanol is present in amounts of about 20 to about 70% of the mixture, and more preferably is about 40 to about 50%. The balance of the solvent system is water.

The washing step then is followed by an "elution" step, which typically uses a fluid containing a high percentage of an organic solvent, such as methanol or acetonitrile. The elution solvent is chosen to effectively release the target compounds from the chromatographic sorbent, and into a suitable sample container. The eluting solvent typically has a pH of less than about 8, more preferably a pH of about 3 to about 6, and most preferably about 4.5 to about 5.5. A pH of about 5 is a highly preferred pH for the eluting solvent. Buffers used to adjust the pH of the eluting solvent include, but are not limited to, acetate, citrate, malate, formate, succinate, and the like. Buffer choice depends upon the desired pH. The eluting solvent typically is a mixture of water and methanol. Methanol is present in amounts greater than 70% of the mixture, and more preferably is greater than about 75%, and most preferably is greater than about 80%. The balance of the solvent system is water.

Elution with high concentrations of organic solvent requires that further steps be taken before analysis. In the case of chromatographic analysis (LC or LC-MS), it is preferable for samples to be dissolved in an aqueous-organic mixture rather than a pure organic solvent, such as methanol or acetonitrile. SPE techniques and materials are described in U.S. Patent Nos. 5,368,729; 5,279,742; 5,260,028; 5,242,598; 5,230,806; 5,137,626; and 5,071,565, each of which is incorporated in its entirety. Sorbent materials for SPE columns include, but are not limited to, C18, C8, cation exchange and hydrophilic-lipophilic-balanced copolymer materials. Such SPE columns are commercially available from a variety of commercial sources, including Waters (e.g., Oasis HLB and MCX), Varian (e.g., C18 and C8), and Millipore (e.g., C18, C8, and mixed phase cation - MPC).

The following examples are provided to demonstrate preferred embodiments of the invention. It should be appreciated by those of skill in the art that the techniques disclosed in the following examples represent techniques discovered by the inventors to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

### EXAMPLES

### Instrumentation

All quantitation experiments were carried out on an API4000 (SciEx) triple quadruple mass spectrometer from Applied Biosystems (Foster City, CA) with Turbo ESI source. The system was controlled by Analysis software 1.3.1. The stability and degradation product identification experiments were carried out on Finnigan LTQ (Thermo-Electron) controlled by Xcalibur Software 1.3.

Separation was performed on a Polaris C18A, 5 µm column (2.1 x 50 mm, Varian). The flow rate was set to 300 µl/min. Elution solvent A was of 5:95 methanol: water (v/v), and solvent B was 95:5 methanol: water, both containing 0.1% formic acid. Two different chromatographic methods were developed. For the quantitative analysis and hepcidin stability study, the HPLC system was composed of a LEAP Technologies (Carrboro, NC) HTS PAL autosampler and a Rheos binary pump. The gradient conditions were set as follows: 0-0.1 min, isocratic 2% B/98% A; 2% B to 95% B at 0.1-4.5 min; 95% B at 4.5-4.9 min; 95% B to 2% B at 4.9-5.0 min; 5.0-6.0 min, isocratic 2% B. The needle wash solvent was methanol : water (50:50, v/v). For the hepcidin enzymatic degradation product identification, the HPLC system consisted of a fully equipped Agilent 1100 configuration (Agilient Technologies, Palo Alto, CA) with temperature-controlled autosampler set at 4 °C. A linear gradient ran from 5% to 95% B in 18 min, then maintained at 95% B for 2 min after which the gradient returned to the initial conditions and equilibrated for 10 min before next run. The sample injection volume was 20 µl for both all the experiments.

### Preparation of Standard Solutions

Stock solution of human hepcidin (SEQ. ID NO: 4) with concentration 1 mg/mL was prepared in 10 mM sodium acetate buffer (pH about 5). One mg/ml stock solution of interval standard (SEQ. ID NO: 17) was prepared in water. SEQ. ID NO: 17 has a sequence of Ac-Lys-Lys-Arg-Pro-Hyp-Gly-CpG-Ser-DTic-CpG, wherein Ac is acetyl, Lys is lysine, Arg is arginine, Pro is proline, Hyp is trans 4-hydroxy-proline, Gly is glycine, CpG is cyclopentylglycine, Ser is serine, and DTic is D-1,2,3,4-tetahydroisoquinoline-3-carboxylic acid. Both stock solutions were stored at -70°C. Final concentration of 100 µg/ml hepcidin working solution was prepared by diluting stock solution with 10 mM sodium acetate buffer. Final internal standard solution with concentration 100 ng/ml was prepared by diluting internal standard stock solution in water.

Calibration standards were prepared freshly by spiking blank serum with hepcidin (SEQ. ID NO: 4) working solution, resulting in concentrations of 10, 25, 50, 100, 250, 500 and 1000 ng/mL of serum. Three different concentrations (20, 200, 800 ng/mL) of quality control (QC) samples were prepared along with the calibration standards.

### Sample Preparation for Stability and Degradation Product Identification.

A human hepcidin stability study was carried out by incubating hepcidin (SEQ. ID NO: 4) in 10% human serum water at room temperature. The incubation was started by mixing 20 µL hepcidin working solution (100 µg/mL) with 1980 µL 10% serum water in a 5 ml test tube, resulting 1 µg/mL initial concentration. A 100 µL solution was aliquoted from the incubation solution at time points of 0h, 0.5h, 1h, 1.5h, 2h, 3h, 4h and 5h. Each aliquot was extracted by SPE immediately and stored at 4 °C until analysis.

### Solid-Phase Extraction

Solid phase extraction was carried out on Oasis HLB 96-well plate (Waters, Milford, MA). Washing solvent: 30% methanol/water with pH of about 10 adjusted with ammonium hydroxide. Elution solvent: 90% methanol/water solution with pH of about 5 adjusted with acetic acid. After activating and conditioning, 100 µL serum sample and 200 µL internal standard were loaded onto the SPE plate, washed with water and 350 µL washing solvent. Elution was done using 100 µL elution solvent and diluted with 100 µL water. The resulting 200 µL eluate was ready for LC-MS analysis.

### ESI-MS/MS Optimization.

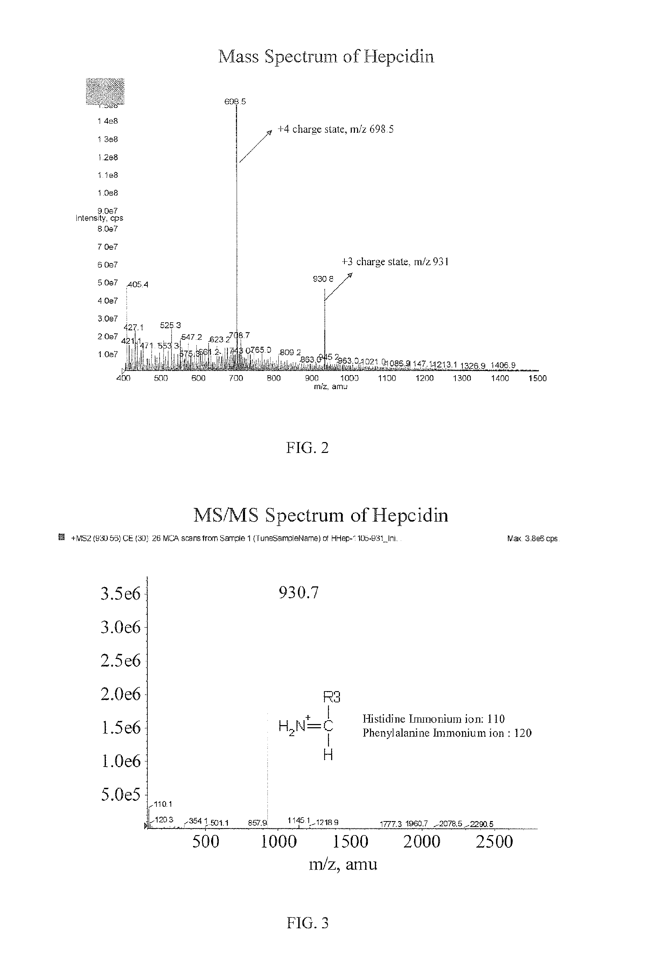

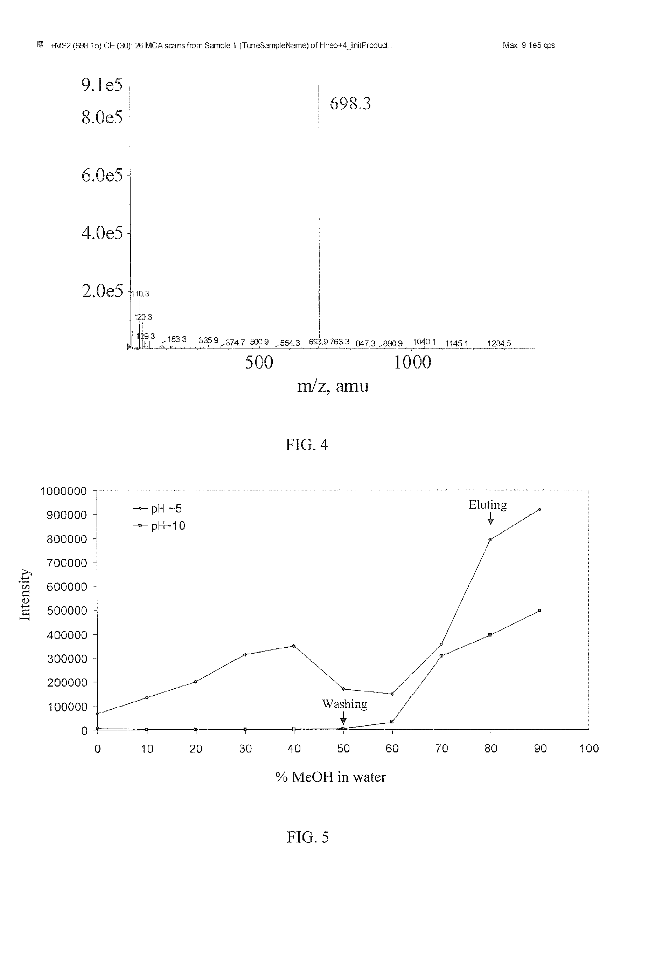

Precursor and product ions for human hepcidin (H-hep; SEQ. ID NO: 4) were determined by direct infusion of 10 µg/mL hepcidin in 50:50 methanol: water (v/v) containing 0.1% formic acid. Since hepcidin is a basic peptide, a positive ionization mode by ESI was used. As shown in FIG. 2, the formation of hepcidin ions with charge state +4 (*m*/*z* 698.5) and +3 (*m*/*z* 930.8) is predominant. After charge de-convolution, an average mass of 2788, which is 8 Da less that the calculated mass from hepcidin sequence, indicates four disulfide bonds formed between the eight cysteine amino acids. FIG. 3 and 4 show the MS/MS spectra for both charge states. The collision assisted dissociation of the hepcidin precursor ions at *m*/*z* 698 and *m*/*z* 931 produce major product ions at *m*/*z* 110 corresponding to the histidine immonium ion and at *m*/*z* 120 corresponding to the phenylalanine ammonium ion. ESI-MS/MS parameters for ion transitions leading to *m*/*z* 110 product ions were optimized for hepcidin quantization as shown in Table II.

**Table II - MS Method**

| Compound | Q1 | Q³ | | Time (msec) | | DP CE | | | CXP |
|---|---|---|---|---|---|---|---|---|---|
| H-hep | 698.10 | 110.15 | | 150 | | 71 | 75 | | 8 |
| H-hep | 930.60 | 110.15 | | 150 | | 81 | 117 | | 8 |
| Internal Standard | 619.10 | 132.15 | | 150 | | 86 | 59 | | 10 |
| CAD | CUR | GS1 | GS2 | IS | TEM | Q1 RES. | | Q3 RES. | |
| 8 | 20 | 20 | 20 | 4000 | 450 | Unit | | Low | |

| | | | | | | | | | |
|---|---|---|---|---|---|---|---|---|---|
| Q1 - quadrupole 1; Q3 - quadrupole 3; DP - declusting potential; CE - collision energy; CXP - collision offset; CAD - collision gas; CUR - curtain gas; GS1 - ion source gas 1; GS2 - ion source gas 2; IS - ion spray voltage; TEM - temperature; Q1 RES - quadrupole 1 resolution; Q3 RES - quadrupole resolution 3 | | | | | | | | | |

### Solid Phase Extraction Optimization for Hepcidin Quantification

Even though tandem mass spectrometry (MS/MS) offers great specificity for detection of analyte of interest, the competition for ionization in the LC-MS interface from co-eluted interfering components of the LC separation can cause significant and inconsistent matrix effect leading to undesirable effects, such as ion suppression and chemical noises. Thus, sample cleanup prior LC separation has a large impact on LC-MS/MS bioanalysis due to the complexity of biological matrices and the limited resolving power of HPLC, especially for fast LC separation. Solid phase extraction is used for hepcidin bioanalysis by which hepcidin is extracted and pre-concentrated, while the amount of the interfering components is greatly reduced.

Solid phase extraction of hepcidin from human serum was performed on a Waters Oasis HLB plate. The sorbent was a hydrophilic/lipophilic balanced polymer which has higher retentivity and capacity than C18. Because hepcidin is a basic peptide, extraction performance is greatly influenced by the pH as well as the organic concentration of the wash and elution solvents. FIG. 5 shows the elution curves of fixed amount of hepcidin using methanol/ water solvents with pH of about 10 adjusted by ammonium hydroxide and solvents with pH about 5 adjusted by acetic acid. Leaching out of hepcidin from the SPE sorbent occurs even with water in acidic condition. In contrast, a maximum of 50% methanol in basic condition does not cause elution of hepcidin from the SPE sorbent. A 30% methanol with pH about 10 was chosen for washing and 90% methanol with pH about 5 was used for hepcidin SPE elution.

### LC-MS/MS of Human hepcidin in serum extract

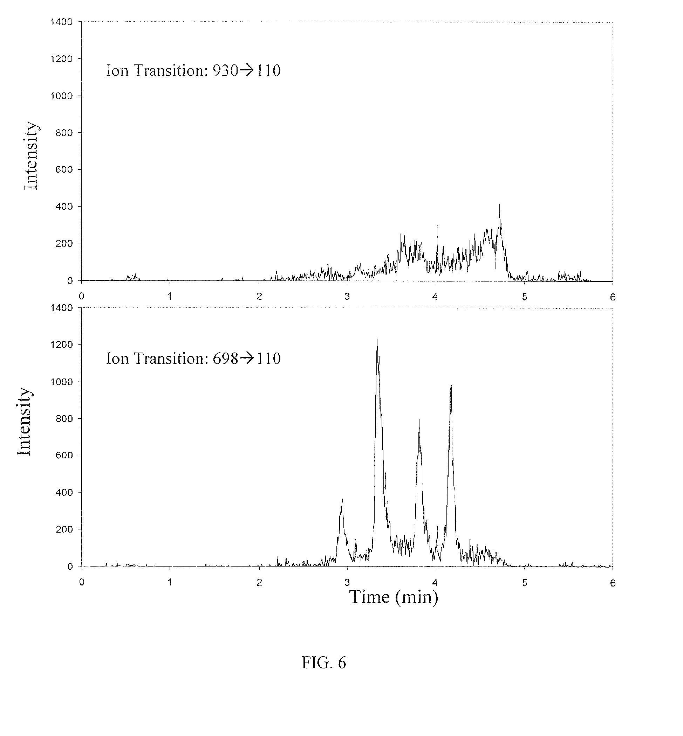

FIG. 5 shows a MRM chromatogram of human hepcidin extracted from blank human serum. Ion transition of *m*/*z* 930.60 → 110.15 gives much cleaner background and less interference from the serum matrix than the ion transition *m*/*z* 698.10 → 110.15, so it was chosen for hepcidin quantification. FIG. 6 shows the chromatogram of hepcidin and internal standard extracted from sepsis patient serum.

### Calibration Curve characteristics, accuracy and precision

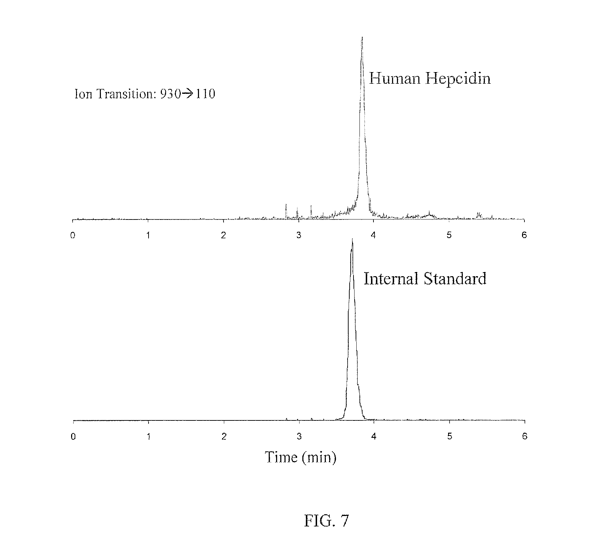

A linear calibration curve was obtained for human hepcidin in serum in the range of 10 to 100 ng/mL, deviations for all seven non-zero standards (10, 25, 50,100, 200, 500 and 1000 ng/mL) in duplicates are all within 15% as shown in FIG. 7.

Accuracy, expressed as the percentage of deviation of the calculated to the nominal concentration (%RE), was evaluated at three concentrations: QC1 (20 ng/mL), QC2 (200 ng/mL) and QC3 (800 ng/mL). All three quality control samples have deviations less than 10%. Precision was also evaluated at three QCs, and all the %RSD values were less than 10% as shown in Table III.

**Table III**

| QC (ng/ml) | Run 1 | Run 2 | Run 3 | Run 4 | Run 5 | Run 6 | AVG (ng/ml) | %RE | %RSD |
|---|---|---|---|---|---|---|---|---|---|
| 20.0 | 19.2 | 16.1 | 17.9 | 19.3 | 21.3 | 18.2 | 18.7 | 6.7 | 9.3 |
| 200.0 | 187.0 | 185.0 | 195.0 | 196.0 | 187.0 | 195.0 | 190.8 | 4.6 | 2.6 |
| 800.0 | 794.0 | 791.0 | 806.0 | 808.0 | 772.0 | 781.0 | 792.0 | 1.0 | 1.8 |

The method was applied to assay hepcidin levels in sepsis patient serum and anemia patient samples from donors. Table IV shows the results of hepcidin level of sepsis patient sera.

**Table IV**

| Sepsis Sample | 1 | 2 | 3 | 4 | 5 | 6 | AVG | %RSD |
|---|---|---|---|---|---|---|---|---|
| 1 | 135 | 152 | 133 | 158 | 133 | 137 | 141.3 | 7.7 |
| 2 | 194 | 222 | 199 | 218 | 203 | 209 | 207.5 | 5.3 |
| 3 | 204 | 213 | 187 | 197 | 190 | 181 | 195.3 | 6.0 |
| 4 | <10 ng/ml | <10 ng/ml | <10 ng/ml | <10 ng/ml | <10 ng/ml | <10 ng/ml | | |
| 5 | <10 ng/ml | <10ng/ml | <10 ng/ml | <10 ng/ml | <10 ng/ml | <10 ng/ml | | |

### Human Hepcidin Stability

Peptides are known to be susceptible to degradation by the endogenous enzymes within the biological matrices. In addition to the 25 peptide human hepcidin, an N-terminally truncated hepcidin-20 and hepcidin-22 also have been reported to exist in human urine. Thus, evaluation of hepcidin stability and its degradation product identification is important in method development. Hepcidin stability was assessed by incubating hepcidin in 10% human serum/water at room temperature with an initial concentration of 1 µg/ml, followed by LC-MS/MS analysis. The ratio of the hepcidin peak area to the internal standard was used to evaluate the stability, and all data were normalized to the value at time point 0h. The stability data (FIG. 8) indicated that human hepcidin are relatively stable. Within five hours incubation, hepcidin showed less than 10% degradation.

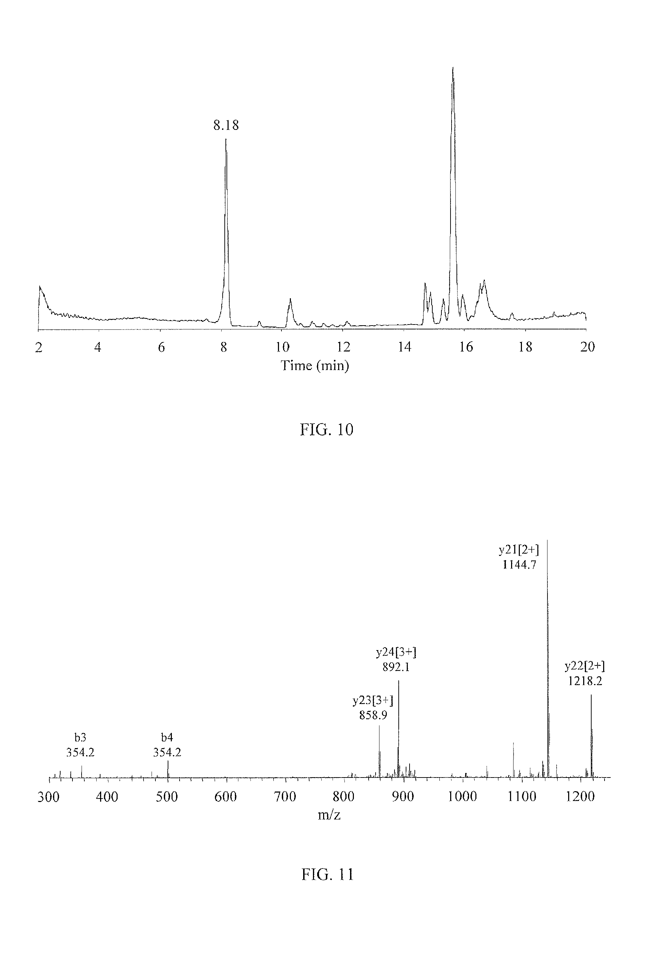

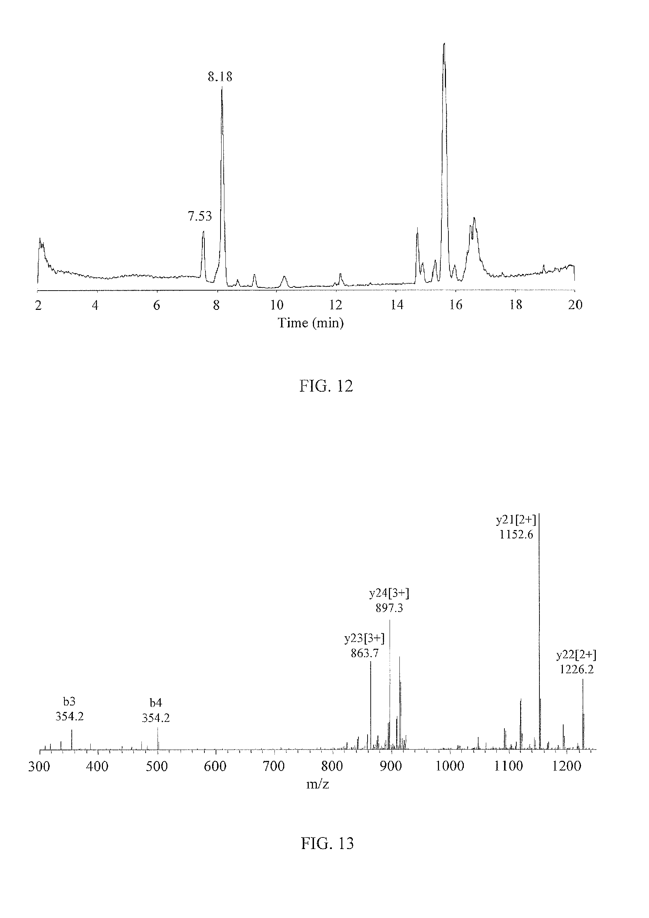

The degradation product was identified by data dependent LC-MS/MS method using Finnigan LTQ instrument. FIG. 9 shows the base peak chromatogram of the incubation solution at 0h time point. Peptide sequencing based on the tandem mass spectrum confirms that the peak at retention time 8.18 min is human hepcidin (FIG. 10). Due to the four internal cysteine disulfide bonds, the major fragment ions are small b ions (b3, b4) and large y ions (y21, y22, y23 and y24). FIG. 11 shows the base peak chromatogram of the solution after four hours incubation. A new peak at retention time of 7.53 min was detected and identified as the human hepcidin with an oxidized methionine (M21) by it MS/MS (FIG. 12).

### Hepcidin Level Measurements of Patients

Samples from patients suffering from anemia of cancer (obtained from ProteoCenex) or volunteers (control) were collected. 100 µL of each sample, serum blanks and calibration standards consisting of seven non-zero concentrations in duplicates (10, 25, 50, 100, 250, 500, 1000 ng/mL) were extracted by SPE using an Oasis HLB mElution 96-well plate (Waters, Milford, MA). Washing solvent was 30% methanol/water with a pH of about 10 adjusted with ammonium hydroxide. Elution solvent was 90% methanol/water solution with a pH of about 5 adjusted with acetic acid. The SPE plate was activated with 500 µL methanol and conditioned with 500 µL water, then 100 µL serum sample and 200 µL internal standard were loaded onto the elution plate, washed with 350 µL water and 350 µL washing solvent. Elution was done using 100 µL elution solvent and diluted with 100 µL water. The resulting 200 µL eluate was analyzed by LC-MS/MS.

20 µl of each extracted sample was injected onto a Polaris C18A, 5 µm HPLC column (2.1 x 50 mm, Varian). The LC flow rate was set to 300 µl/min. The HPLC mobile phase A was 5:95 methanol/water, and mobile phase B was 95:5 methanol/water, both containing 0.1% formic acid. The gradient conditions were set as follows: 0-0.1 min, isocratic 2% B/98% A; 2% B to 95% B at 0-1-4.5 min; 95%B at 4.5-4.9 min; 95% B to 2%B at 4.9-5.0 min; 5.0-6.0 min, isocratic 2% B.

A Sciex AP14000 triple quadrupole mass spectrometer from Applied Biosystems (Foster City, CA) with Turbo ESI source was used for hepcidin detection in MRM mode with ion transition of *m*/*z* 930.60 to *m*/*z* 110.15. Quantification was achieved by comparing the ratio of the LC peak areas of the hepcidin and the internal standard to the ratios obtained from a series of standards where the amounts of hepcidin and internal standard were known.

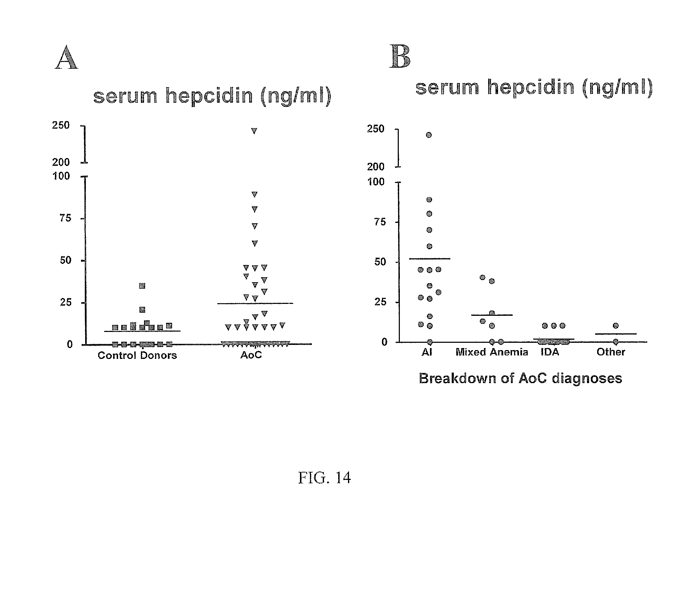

This experiment allowed for the determination of the serum levels of hepcidin in a control population presumed to contain a large number of healthy individuals, as well as the serum level of hepcidin from patients suffering anemia of cancers (AOC). The results are shown in FIG. 14A.

After identification of elevated hepcidin levels, a sample was then analyzed for other iron index concentrations to determine whether a patient had inflammation or iron deficiency anemia (FIG. 14B). The parameters were measured as follows: serum iron, UIBC, ferritin, and CRP were measured on an Olympus AU400 clinical laboratory analyzer using standard procedures; sTfR was measured using a standard ELISA method (R&D systems).

### Separation and Determination of Hepcidin-20, Hepcidin-22, and Hepcidin-25 in a Sample

The relative and/or absolute levels of hepcidin-20, hepcidin-22, and hepcidin-25 in a sample may be relevant to the biological activity to be expected. Hepcidin-20 and hepcidin 22 are believed to be breakdown products of the full length mature peptide. Hepcidin-20 has been demonstrated to be inactive in a biological assay, presumably due to the inability to bind ferroportin. Hence, hepcidin-20 and potentially hepcidin-22 may be inactive constituents of the circulating hepcidin pool. Since in certain disease states such as chronic kidney disease the normal clearance of hepcidin may be affected, the relative abundance of different forms may be affected. For this reason, there are advantages to developing detection methods which allow estimation of full length material (hepcidin -25) relative to hepcidin-22 and hepcidin -20.

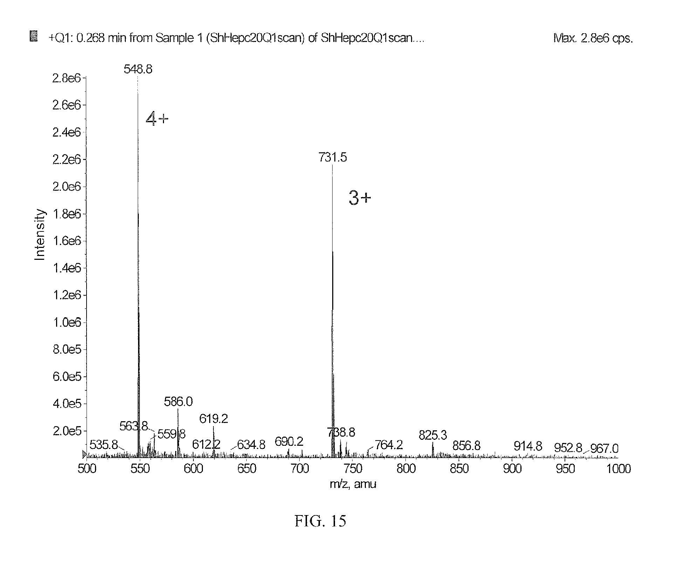

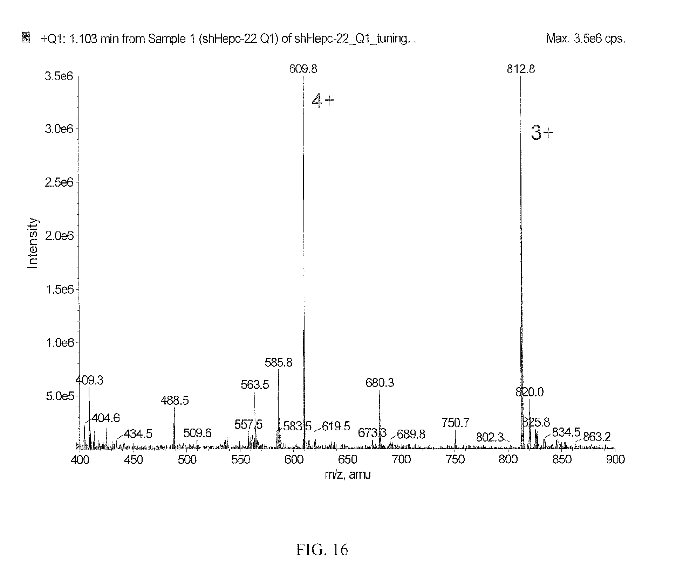

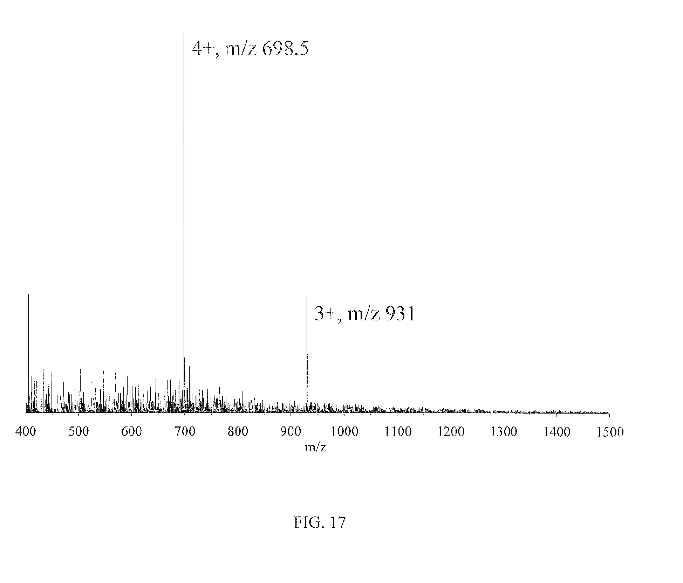

Precursor and product ions for N-terminal truncated human hepcidin Hepc-20 (SEQ ID NO: 15), Hepc-22 (SEQ ID NO: 16) and intact Hepc-25 (SEQ ID NO: 4) were determined by direct infusion of 10 µg/ml hepcidin in 50:50 methanol : water (v/v) containing 0.1% formic acid. A positive ionization mode by ESI was used. As shown in FIG. 15-17, the formation of charge states of 4+ and 3+ ions for the three hepcidin peptides is predominant.

The collision assisted dissociation of the Hepc-20, Hepc-22 and Hepc-25 precursor ions produced unique product ions which were optimized and used for the quantitation of Hepc-20, Hepc-22 and Hepc-25 as shown in Table V. Isotope labeled Hepcidin Hepc-25* was used as internal standard.

**Table V**

| Compound | Q1 | Q3 | Time (msec) | DP | CE | CXP |
|---|---|---|---|---|---|---|
| Hepc-25* IS | 700.58 | 354.05 | 150 | 76 | 41 | 26 |
| Hepc-22 | 610.01 | 763.95 | 150 | 41 | 27 | 12 |
| Hepc-20 | 548.88 | 693.55 | 150 | 36 | 21 | 10 |
| Hepc-25d | 698.08 | 354.05 | 150 | 76 | 41 | 26 |
| CUR | GS1 | GS2 | IS | TEM | CAD | |
| 40 | 70 | 50 | 5000 | 450 | 10 | |

| | | | | | | |
|---|---|---|---|---|---|---|
| Q1 - quadrupole 1; Q3 - quadrupole 3; DP - declusting potential; CE - collision energy; CXP - collision offset; CUR - curtain gas; GS1 - ion source gas 1; GS2 - ion source gas 2; IS - ion spray voltage; Term - temperature; CAD - collision gas; | | | | | | |

Separation of the three hepcidins in each sample was performed on a Polaris C18A, 5 µm column (75 x 2.0 mm, Varian). The flow rate was set to 400 µl/min. Elution solvent A was 5:95 methanol : water (v/v), and solvent B was 95:5 methanol: water, both containing 0.1% formic acid. The HPLC system was composed of a LEAP Technologies (Carrboro, NC) HTS PAL autosampler and a Rheos binary pump. The gradient conditions were set as follows: 0-0.5 min, isocratic 2% B/98% A; 2% B to 95% B at 0.5-4.0 min; 95% B at 4.0-5.4 min; 95% B to 2% B at 5.4-5.5 min; 5.5-6.5 min, isocratic 2% B. The sample injection volume was 20 µl for all the experiments.

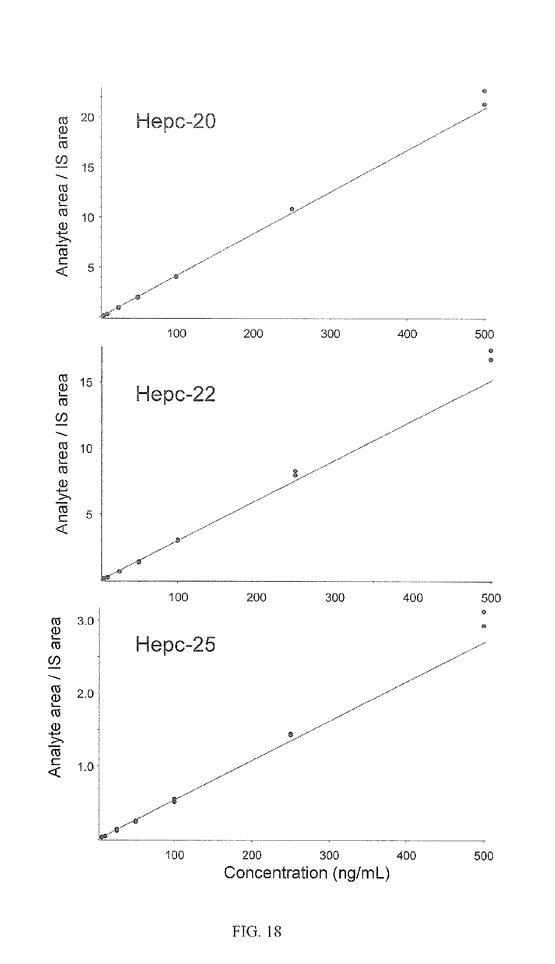

Calibration standards were prepared freshly by spiking blank serum with human hepcidin peptides, resulting in concentrations of 2.5, 5, 10, 25, 50, 100,250, 500 ng/ml of Hepc-20, Hepc-22 and Hepc-25 in serum standards. Both Hepc-20 and Hepc-22 showed excellent linear range of 2.5 to 500 ng/mL, while Hepc-25 showed excellent linear range of 5 to 500 ng/mL (FIG. 18).

Solid phase extraction was carried out on Oasis HLB 96-well plate (Waters, Milford, MA). The washing solvent was 30% methanol in water at pH 10 adjusted with ammonium hydroxide. The elution solvent was 90% methanol in water solution at pH 5 adjusted with acetic acid. After activating and conditioning, 100 µl serum samples and 200 µl internal standard were loaded onto the SPE plate, then washed with water and 350 µl washing solvent. Elution was performed using 100 µl elution solvent and diluted with 100 µl water. The resulting 200 µl elute was ready for LC-MS analysis.

Nine sepsis samples were analyzed using the above protocol. The concentrations of Hepc-20, Hepc-22, and Hepc-25 were determined using the calibration curve, with BQL indicating that the level was below the quantifiable level (either 5 ng/mL for Hepc-25 or 2.5 ng/mL for Hepc-20 and Hepc-22). The results are shown below in Table VI.

**Table VI**

| **Sepsis sample** | **Hepc-25** | **Hepc-22** | **Hepc-20** |
|---|---|---|---|
| Human serum blank | BQL | BQL | BQL |
| Human serum blank | BQL | BQL | BQL |
| Human serum blank | BQL | BQL | BQL |
| 1 | 1260 | 33.4 | 68.4 |
| 2 | 1220 | 34.9 | 78.7 |
| 3 | 350 | 5.59 | 11.2 |
| 4 | 18.2 | BQL | BQL |
| 5 | 26.5 | BQL | BQL |
| 6 | 20 | 2.82 | 16.3 |
| 7 | 790 | 7.22 | 105 |
| 8 | 216 | 6.25 | 40.1 |
| 9 | BQL | BQL | BQL |
| | | | |
| | BQL: <LLOQ=5 ng/mL | BQL:<LLOQ=2.5ng/mL | BQL: <LLOQ=2.5 ng/mL |

Sepsis patients were used for the analysis due to their high hepcidin levels, allowing accurate quantitation of less abundant hepcidin species such as hepcidin-22 and hepcidin-20. These results demonstrate that accurate detection of the less abundant forms can be observed using this assay. Hence, utilization of this method will allow assessment of the ratio of active hepcidin to total hepcidin in disease populations.

The invention also relates to the following aspects as defined in the following numbered paragraphs:
1. A method of determining a presence or concentration of hepcidin in a sample comprising subjecting the sample to mass spectrometry to produce a mass spectrum having a hepcidin signal; measuring an intensity of the hepcidin signal; and correlating the signal intensity to a standard curve of hepcidin concentrations to determine the presence or concentration of the hepcidin in the sample.
2. The method of paragraph 1, wherein the hepcidin comprises about 84 amino acid residues.
3. The method of paragraph 1, wherein the hepcidin comprises about 61 amino acid residues.
4. The method of paragraph 1, wherein the hepcidin comprises about 20 to about 25 amino acid residues.
5. The method of paragraph 1, wherein the hepcidin comprises an amino acid sequence selected from the group consisting of: SEQ ID NO: 3; SEQ ID NO: 4; SEQ ID NO: 13; SEQ ID NO: 14; SEQ ID NO: 15; and any combination(s) thereof.
6. The method of paragraph 1, wherein the hepcidin has a +4 or +3 charge.
7. The method of paragraph 1, wherein the hepcidin has a +2 or +1 charge.
8. The method of paragraph 1, further comprising separating the hepcidin from the sample prior to subjecting the hepcidin to mass spectrometry.
9. The method of paragraph 8, wherein the separating comprises chromatography.
10. The method of paragraph 9, wherein the chromatography is liquid chromatography.
11. The method of paragraph 9, wherein the chromatography is a solid phase extraction.
12. The method of paragraph 1, wherein the mass spectrometry comprises analyzing by liquid chromatography-mass spectrometry.
13. The method of paragraph 1, wherein the mass spectrometry comprises tandem mass spectrometry.
14. A method of determining the presence or concentration of hepcidin in a sample comprising separating the hepcidin from the sample; subjecting the hepcidin to mass spectrometry to produce a mass spectrum having a hepcidin signal; measuring the intensity of the hepcidin signal; and correlating the signal intensity in the mass spectrum to a standard curve of hepcidin concentrations to obtain a quantity of hepcidin in the sample.
15. The method of paragraph 14, wherein the separating comprises chromatography.
16. The method of paragraph 15, wherein the chromatography is liquid chromatography.
17. The method of paragraph 15, wherein the chromatography is a solid phase extraction.
18. The method of paragraph 14, wherein the hepcidin comprises about 84 amino acid residues.
19. The method of paragraph 14, wherein the hepcidin comprises about 20 to about 25 amino acid residues.
20. The method of paragraph 14, wherein the hepcidin has a +4 or a +3 charge.
21. The method of paragraph 14, wherein the hepcidin has a +2 or a +1 charge.
22. A method of separating hepcidin from a sample comprising introducing the sample to a reverse phase column and treating the reverse phase column with an eluting solvent, wherein a resulting eluant comprises hepcidin.
23. The method of paragraph 22, wherein the eluting solvent comprises methanol, water, or mixtures thereof.
24. The method of paragraph 23, wherein the methanol concentration is about 20 to about 50% by volume.
25. The method of paragraph 22, wherein the reverse phase column is selected from the group consisting of C3, C8, C18, and combination thereof.
26. The method of paragraph 1, 14, or 22, wherein the sample is a plasma sample or a serum sample.
27. The method of paragraph 1, 14, 22, or 26 wherein the sample is from a mammal.
28. The method of paragraph 27, wherein the mammal is a human.
29. The method of paragraph 28, wherein the human is not suffering from sepsis or an inflammatory condition.
30. The method of paragraph 28, wherein iron homeostasis is disrupted.
31. The method of paragraph 30, wherein the iron homeostasis is below normal.
32. The method of paragraph 30, wherein the iron homeostasis is above normal.
33. The method of paragraph 30, wherein one or more iron indices is greater than a range listed in Table I.
34. The method of paragraph 30, wherein one or more iron indices is outside a range listed in Table I.
35. The method of paragraph 28, wherein the human suffers from or is suspected of suffering from an inflammatory or inflammatory-related condition.
36. The method of paragraph 35, wherein the condition is selected from the group consisting of sepsis, anemia of inflammation, anemia of cancer, chronic inflammatory anemia, congestive heart failure, end stage renal disorder, chronic kidney disease, iron deficiency anemia, ferroportin disease, hemochromatosis, diabetes, rheumatoid arthritis, arteriosclerosis, tumors, vasculitis, systemic lupus erythematosus, hemoglobinopathies, red cell disorders, and kidney failure.
37. The method of paragraph 28, wherein the human suffers from or is suspected of suffering from a non-inflammatory condition.
38. The method of paragraph 37, wherein the non-inflammatory condition is selected from the group consisting of vitamin B6 deficiency, vitamin B12 deficiency, folate deficiency, pellagra, funicular myelosis, pseudoencephalitis, Parkinson's disease, Alzheimer's disease, coronary heart disease, and peripheral occlusive arterial disease.
39. The method of paragraph 28, wherein the human suffers from or is suspected of suffering from an acute phase reaction.
40. The method of paragraph 39, wherein the acute phase reaction is selected from the group consisting of sepsis, pancreatitis, hepatitis, and rheumatoid diseases.
41. The method of paragraph 28, wherein the human is hypo-responsive to erythropoietin therapy or an erythropoietic therapy.
42. The method of paragraph 41, wherein the erythropoietic therapy is darbepoetin alfa.
43. In a method of treating a patient suffering from anemia, the improvement comprising determination of a hepcidin concentration in a sample from the patient, wherein the determination comprises separating the hepcidin from the sample; subjecting the hepcidin to mass spectrometry to produce a mass spectrum having a hepcidin signal; measuring the intensity of the hepcidin signal; and correlating the signal intensity in the mass spectrum to a standard curve of hepcidin concentrations to obtain a quantity of hepcidin in the sample.
44. The method of paragraph 43, wherein the hepcidin concentration is about 10 ng/mL or less.
45. The method of paragraph 43, wherein the hepcidin concentration is about 5 ng/mL or less.
46. The method of paragraph 43, wherein the hepcidin concentration is about 2 ng/mL or less.
47. The method of paragraph 43, wherein the hepcidin concentration is about 1 ng/mL or less.
48. The method of paragraph 43, wherein the hepcidin concentration is about 30 ng/mL or greater.
49. The method of paragraph 43, wherein the hepcidin concentration is about 50 ng/mL or greater.
50. A kit comprising:
   a) a plurality of containers, each container comprising a known and different amount of a Hepcidin;
   b) a container comprising a standard for admixture with each container of (a) and admixture with a test sample;
      and c) instructions for (1) preparing a standard curve from (a) and (b) and (2) assaying the test sample for a hepcidin's concentration in the test sample using the standard curve.
51. The kit of paragraph 50, wherein the standard comprises an isotopically labeled standard.
52. The kit of paragraph 51, wherein the standard comprises SEQ ID NO: 4.
53. The kit of paragraph 52, wherein the hepcidin comprises SEQ ID NO: 4.

## Claims

1. A method of determining a presence or concentration of hepcidin in a sample comprising subjecting the sample to mass spectrometry to produce a mass spectrum having a hepcidin signal; measuring an intensity of the hepcidin signal; and correlating the signal intensity to a standard curve of hepcidin concentrations to determine the presence or concentration of the hepcidin in the sample.

2. The method of claim 1, wherein the hepcidin comprises about 20 to about 25 amino acid residues.

3. The method of claim 1, wherein the hepcidin comprises an amino acid sequence selected from the group consisting of: SEQ ID NO: 3; SEQ ID NO: 4; SEQ ID NO: 13; SEQ ID NO: 14; SEQ ID NO: 15; and any combination(s) thereof.

4. The method of claim 1, further comprising separating the hepcidin from the sample prior to subjecting the hepcidin to mass spectrometry.

5. A method of determining the presence or concentration of hepcidin in a sample comprising separating the hepcidin from the sample; subjecting the hepcidin to mass spectrometry to produce a mass spectrum having a hepcidin signal; measuring the intensity of the hepcidin signal; and correlating the signal intensity in the mass spectrum to a standard curve of hepcidin concentrations to obtain a quantity of hepcidin in the sample.

6. The method of claim 5, wherein the separating comprises chromatography.

7. A method of separating hepcidin from a sample comprising introducing the sample to a reverse phase column and treating the reverse phase column with an eluting solvent, wherein a resulting eluant comprises hepcidin, wherein the eluting solvent comprises methanol, water, or mixtures thereof.

8. The method of claim 1, 5, or 7, wherein the sample is a plasma sample or a serum sample from a mammal, and preferably a human.

9. The method of claim 8, wherein the human suffers from or is suspected of suffering from an inflammatory or inflammatory-related condition.

10. The method of claim 9, wherein the condition is selected from the group consisting of sepsis, anemia of inflammation, anemia of cancer, chronic inflammatory anemia, congestive heart failure, end stage renal disorder, chronic kidney disease, iron deficiency anemia, ferroportin disease, hemochromatosis, diabetes rheumatoid arthritis, arteriosclerosis, tumors, vasculitis, systemic lupus erythematosus, hemoglobinopathies, red cell disorders, and kidney failure.

11. The method of claim 8, wherein the human suffers from or is suspected of suffering from a non-inflammatory condition.

12. The method of claim 11, wherein the non-inflammatory condition is selected from the group consisting of vitamin B6 deficiency, vitamin B12 deficiency, folate deficiency, pellagra, funicular myelosis, pseudoencephalitis, Parkinson's disease, Alzheimer's disease, coronary heart disease, and peripheral occlusive arterial disease.

13. The method of claim 8, wherein the human suffers from or is suspected of suffering from an acute phase reaction.

14. The method of claim 8, wherein the human is hypo-responsive to erythropoietin therapy or an erythropoietic therapy.

15. A method for determining a hepcidin concentration in a sample from a patient suffering from anemia, comprising separating the hepcidin from the sample; subjecting the hepcidin to mass spectrometry to produce a mass spectrum having a hepcidin signal; measuring the intensity of the hepcidin signal; and correlating the signal intensity in the mass spectrum to a standard curve of hepcidin concentrations to obtain a quantity of hepcidin in the sample.

16. The method of claim 15, wherein the hepcidin concentration is about 10 ng/mL or less, preferably about 5 ng/mL or less, and more preferably about 1 ng/mL or less.

17. The method of claim 15, wherein the hepcidin concentration is about 30 ng/mL or greater or about 50 ng/mL or greater.

18. A kit comprising:
a) a plurality of containers, each container comprising a known and different amount of a hepcidin;
b) a container comprising a standard for admixture with each container of (a) and admixture with a test sample; and
c) instructions for (1) preparing a standard curve from (a) and (b) and (2) assaying the test sample for a hepcidin's concentration in the test sample using the standard curve.


SEQUENCE LISTING

<110>  Li, et al.

<120>  Method of Detecting And/Or Measuring Hepcidin in a Sample

<130>  01017/41927A

<150>  60/832,625
<151>  2006-07-21

<160>  19

<170>  PatentIn version 3.3

<210>  1
<211>  83
<212>  PRT
<213>  Mus musculus

<400>  1

Met Ala Leu Ser Thr Arg Thr Gln Ala Ala Cys Leu Leu Leu Leu Leu
1               5                   10                  15

Leu Ala Ser Leu Ser Ser Thr Thr Tyr Leu His Gln Gln Met Arg Gln
            20                  25                  30

Thr Thr Glu Leu Gln Pro Leu His Gly Glu Glu Ser Arg Ala Asp Ile
        35                  40                  45

Ala Ile Pro Met Gln Lys Arg Arg Lys Arg Asp Thr Asn Phe Pro Ile
    50                  55                  60

Cys Ile Phe Cys Cys Lys Cys Cys Asn Asn Ser Gln Cys Gly Ile Cys
65                  70                  75                  80

Cys Lys Thr

<210>  2
<211>  84
<212>  PRT
<213>  Rattus norvegicus

<400>  2

Met Ala Leu Ser Thr Arg Ile Gln Ala Ala Cys Leu Leu Leu Leu Leu
1               5                   10                  15

Leu Ala Ser Leu Ser Ser Gly Ala Tyr Leu Arg Gln Gln Thr Arg Gln
            20                  25                  30

Thr Thr Ala Leu Gln Pro Trp His Gly Ala Glu Ser Lys Thr Asp Asp
        35                  40                  45

Ser Ala Leu Leu Met Leu Lys Arg Arg Lys Arg Asp Thr Asn Phe Pro
    50                  55                  60

Ile Cys Leu Phe Cys Cys Lys Cys Cys Lys Asn Ser Ser Cys Gly Leu
65                  70                  75                  80

Cys Cys Ile Thr

<210>  3
<211>  84
<212>  PRT
<213>  Homo sapiens

<400>  3

Met Ala Leu Ser Ser Gln Ile Trp Ala Ala Cys Leu Leu Leu Leu Leu
1               5                   10                  15

Leu Leu Ala Ser Leu Thr Ser Gly Ser Val Phe Pro Gln Gln Thr Gly
            20                  25                  30

Gln Leu Ala Glu Leu Gln Pro Gln Asp Arg Ala Gly Ala Arg Ala Ser
        35                  40                  45

Trp Met Pro Met Phe Gln Arg Arg Arg Arg Arg Asp Thr His Phe Pro
    50                  55                  60

Ile Cys Ile Phe Cys Cys Gly Cys Cys His Arg Ser Lys Cys Gly Met
65                  70                  75                  80

Cys Cys Lys Thr

<210>  4
<211>  25
<212>  PRT
<213>  Homo sapiens

<400>  4

Asp Thr His Phe Pro Ile Cys Ile Phe Cys Cys Gly Cys Cys His Arg
1               5                   10                  15

Ser Lys Cys Gly Met Cys Cys Lys Thr
            20                  25

<210>  5
<211>  25
<212>  PRT
<213>  Macaca fascicularis

<400>  5

Asp Thr His Phe Pro Ile Cys Ile Phe Cys Cys Gly Cys Cys His Arg
1               5                   10                  15

Ser Lys Cys Gly Met Cys Cys Arg Thr
            20                  25

<210>  6
<211>  25
<212>  PRT
<213>  Cercopithecus aethiops

<400>  6

Asp Thr His Phe Pro Ile Cys Ile Phe Cys Cys Gly Cys Cys His Arg
1               5                   10                  15

Ser Lys Cys Gly Met Cys Cys Arg Thr
            20                  25

<210>  7
<211>  25
<212>  PRT
<213>  Oryctolagus cuniculus

<400>  7

Asp Thr His Phe Pro Ile Cys Ile Phe Cys Cys Ser Cys Cys Arg Asn
1               5                   10                  15

Ser Lys Cys Gly Ile Cys Cys Lys Thr
            20                  25

<210>  8
<211>  25
<212>  PRT
<213>  Rattus norvegicus

<400>  8

Asp Thr Asn Phe Pro Ile Cys Leu Phe Cys Cys Lys Cys Cys Lys Asn
1               5                   10                  15

Ser Ser Cys Gly Leu Cys Cys Ile Thr
            20                  25

<210>  9
<211>  25
<212>  PRT
<213>  Mus musculus

<400>  9

Asp Thr Asn Phe Pro Ile Cys Ile Phe Cys Cys Lys Cys Cys Asn Asn
1               5                   10                  15

Ser Gln Cys Gly Ile Cys Cys Lys Thr

20                  25


<210>  10
<211>  25
<212>  PRT
<213>  Canis familiaris

<400>  10

Asp Thr His Phe Pro Ile Cys Ile Phe Cys Cys Gly Cys Cys Lys Thr
1               5                   10                  15


Pro Lys Cys Gly Phe Cys Cys Arg Thr
            20                  25


<210>  11
<211>  25
<212>  PRT
<213>  Canis familiaris

<400>  11

Asp Thr His Phe Pro Ile Cys Ile Phe Cys Cys Gly Cys Cys Lys Thr
1               5                   10                  15


Pro Lys Cys Gly Phe Cys Cys Lys Thr
            20                  25


<210>  12
<211>  25
<212>  PRT
<213>  Canis familiaris

<400>  12

Asp Thr His Phe Pro Ile Cys Ile Phe Cys Cys Gly Cys Cys Lys Thr
1               5                   10                  15


Pro Lys Cys Gly Leu Cys Cys Lys Thr
            20                  25


<210>  13
<211>  60
<212>  PRT
<213>  Homo sapiens

<400>  13

Ser Val Phe Pro Gln Gln Thr Gly Gln Leu Ala Glu Leu Gln Pro Gln
1               5                   10                  15


Asp Arg Ala Gly Ala Arg Ala Ser Trp Met Pro Met Phe Gln Arg Arg
            20                  25                  30


Arg Arg Arg Asp Thr His Phe Pro Ile Cys Ile Phe Cys Cys Gly Cys
        35                  40                  45

Cys His Arg Ser Lys Cys Gly Met Cys Cys Lys Thr
    50                  55                  60

<210>  14
<211>  60
<212>  PRT
<213>  Homo sapiens

<400>  14

Ser Val Phe Pro Gln Gln Thr Gly Gln Leu Ala Glu Leu Gln Pro Gln
1               5                   10                  15

Asp Arg Ala Gly Ala Arg Ala Ser Trp Met Pro Met Phe Gln Arg Arg
            20                  25                  30

Arg Arg Arg Asp Thr His Phe Phe Ile Cys Ile Phe Cys Cys Gly Cys
        35                  40                  45

Cys His Arg Ser Lys Cys Gly Met Cys Cys Lys Thr
    50                  55                  60

<210>  15
<211>  20
<212>  PRT
<213>  Homo sapiens

<400>  15

Ile Cys Ile Phe Cys Cys Gly Cys Cys His Arg Ser Lys Cys Gly Met
1               5                   10                  15

Cys Cys Lys Thr
            20

<210>  16
<211>  22
<212>  PRT
<213>  Homo sapiens

<400>  16

Phe Pro Ile Cys Ile Phe Cys Cys Gly Cys Cys His Arg Ser Lys Cys
1               5                   10                  15

Gly Met Cys Cys Lys Thr
            20

<210>  17
<211>  10
<212>  PRT
<213>  Artificial sequence

<220>

<223>  Synthetic peptide

<220>
<221>  acetylation
<222>  (1)..(1)
<223>  residue in position 1 is acetylated

<220>
<221>  misc_feature
<222>  (6)..(6)
<223>  Xaa=trans 4-hydroxy-proline

<220>
<221>  misc_feature
<222>  (8)..(8)
<223>  Xaa=cyclopentylglycine

<220>
<221>  misc_feature
<222>  (10)..(10)
<223>  Xaa=D-1,2,3,4-tetrahydroisoquinoline-3-carboxylic acid

<220>
<221>  misc_feature
<222>  (11)..(11)
<223>  Xaa=cyclopentylglycine

<400>  17

Lys Lys Arg Pro Xaa Gly Xaa Ser Xaa Xaa
1               5                   10

<210>  18
<211>  193
<212>  PRT
<213>  Homo sapiens

<220>
<221>  mat_peptide
<222>  (28)..(192)

<400>  18

Met Gly Val His Glu Cys Pro Ala Trp Leu Trp Leu Leu Leu Ser Leu
        -25                 -20                 -15

Leu Ser Leu Pro Leu Gly Leu Pro Val Leu Gly Ala Pro Pro Arg Leu
    -10                 -5                  -1  1               5

Ile Cys Asp Ser Arg Val Leu Glu Arg Tyr Leu Leu Glu Ala Lys Glu
                10                  15                  20

Ala Glu Asn Ile Thr Thr Gly Cys Ala Glu His Cys Ser Leu Asn Glu
            25                  30                  35

Asn Ile Thr Val Pro Asp Thr Lys Val Asn Phe Tyr Ala Trp Lys Arg
        40                  45                  50

Met Glu Val Gly Gln Gln Ala Val Glu Val Trp Gln Gly Leu Ala Leu
    55                  60                  65

Leu Ser Glu Ala Val Leu Arg Gly Gln Ala Leu Leu Val Asn Ser Ser
70                  75                  80                  85

Gln Pro Trp Glu Pro Leu Gln Leu His Val Asp Lys Ala Val Ser Gly
                90                  95                  100

Leu Arg Ser Leu Thr Thr Leu Leu Arg Ala Leu Gly Ala Gln Lys Glu
            105                 110                 115

Ala Ile Ser Pro Pro Asp Ala Ala Ser Ala Ala Pro Leu Arg Thr Ile
        120                 125                 130

Thr Ala Asp Thr Phe Arg Lys Leu Phe Arg Val Tyr Ser Asn Phe Leu
    135                 140                 145

Arg Gly Lys Leu Lys Leu Tyr Thr Gly Glu Ala Cys Arg Thr Gly Asp
150                 155                 160                 165

Arg

<210>  19
<211>  193
<212>  PRT
<213>  Homo sapiens

<220>
<221>  mat_peptide
<222>  (28)..(192)

<400>  19

Met Gly Val His Glu Cys Pro Ala Trp Leu Trp Leu Leu Leu Ser Leu
        -25                 -20                 -15

Leu Ser Leu Pro Leu Gly Leu Pro Val Leu Gly Ala Pro Pro Arg Leu
    -10                 -5                  -1  1               5

Ile Cys Asp Ser Arg Val Leu Glu Arg Tyr Leu Leu Glu Ala Lys Glu
                10                  15                  20

Ala Glu Asn Ile Thr Thr Gly Cys Ala Glu His Cys Ser Leu Asn Glu
            25                  30                  35

Asn Ile Thr Val Pro Asp Thr Lys Val Asn Phe Tyr Ala Trp Lys Arg
    40                  45                  50

Met Glu Val Gly Gln Gln Ala Val Glu Val Trp Gln Gly Leu Ala Leu
    55                  60                  65

Leu Ser Glu Ala Val Leu Arg Gly Gln Ala Leu Leu Val Asn Ser Ser
70                  75                  80                  85

Gln Pro Trp Glu Pro Leu Gln Leu His Val Asp Lys Ala Val Ser Gly
                90                  95                  100

Leu Arg Ser Leu Thr Thr Leu Leu Arg Ala Leu Gly Ala Gln Lys Glu
            105                 110                 115

Ala Ile Ser Pro Pro Asp Ala Ala Ser Ala Ala Pro Leu Arg Thr Ile
        120                 125                 130

Thr Ala Asp Thr Phe Glu Lys Leu Phe Arg Val Tyr Ser Asn Phe Leu
    135                 140                 145

Arg Gly Lys Leu Lys Leu Tyr Thr Gly Glu Ala Cys Arg Thr Gly Asp
150                 155                 160                 165

Arg